# FASCICULE DE BREVET EUROPEEN

(11) **EP 1 044 260 B1**
(45) Date de publication et mention de la délivrance du brevet: **14.05.2008**
(21) Numéro de dépôt: 98964559.3
(22) Date de dépôt: 30.12.1998
(51) Int. Cl.: C12F 3/00, C07C 37/82, C07D 311/40, C07H 1/08

(54) **PROCEDE D'OBTENTION D'UN TANIN DE RAISIN, TANIN OBTENU ET UTILISATIONS**
VERFAHREN ZUR GEWINNUNG EINES TRAUBENTANNIN, ERHALTENES TANNIN UND VERWENDUNGEN
METHOD FOR OBTAINING GRAPE TANNIN, RESULTING TANNIN AND USES

(30) Priorité: 30.12.1997 FR 9716860
(43) Date de publication de la demande: 18.10.2000
(73) Titulaire: Ferco, 07220 Saint-Montan (FR)
(72) Inventeur: FERIES, Marc, F-07220 Saint-Montan (FR)
(74) Mandataire: Guerre, Dominique
(86) Numéro de dépôt international: PCT/FR1998/002923
(87) Numéro de publication internationale: WO 1999/035239

(56) Documents cités:
- FR-A- 2 372 823
- FR-A- 2 643 073
- CHEMICAL ABSTRACTS, vol. 88, no. 11, 13 mars 1978 Columbus, Ohio, US; abstract no. 72940w, MANDZHUKOV, B.: "Effect of some factors on the extraction of phenolic compounds from grapes" page 380; XP002075402 & LOZAR. VINAR., vol. 26, no. 5, 1977, pages 15-22,

## Description

La présente invention concerne l'obtention, à partir de raisin frais noir ou blanc, de tanin de raisin de marc ou de pépin, à teneur élevée en composés polyphénoliques, le tanin ainsi obtenu et les utilisations de celui-ci notamment en oenologie.

Les tanins sont des substances d'origine végétale constituées de composés polyphénoliques et que l'on divise en deux groupes principaux, les tanins catéchiques aussi appelés tanins condensés ou procyanidines, auxquels appartiennent les tanins de raisin, et les tanins hydrolysables comprenant majoritairement les gallotanins et les ellagitanins. Les tanins de raisin sont naturellement présents dans les pellicules et les pépins de raisin.

Ces deux groupes se distinguent par la nature des composés polyphénoliques qui les constituent. Les tanins catéchiques rassemblent des dérivés catéchiques et des oligomères et polymères procyanidoliques constitués d'unités flavanol-3 et flavanediol-3,4. Plus particulièrement, les tanins de pépin et de pellicule de raisin sont constitués de catéchine, épicatéchine et épicatéchine-3-O-gallate, le tanin de pellicule contenant en outre de l'épigallocatéchine, absente dans le tanin de pépin. Les polymères du tanin de pellicule sont plus longs (chaînes atteignant 80 unités) que les polymères de tanin de pépin (chaînes ne dépassant pas 30 à 35 unités). Enfin sur le tanin de pépin se greffent des molécules d'acide gallique, que l'on ne retrouve qu'en très faible quantité dans le tanin de pellicule.

Les gallotanins du groupe des tanins hydrolysables sont constitués de polymères du glucose et de l'acide gallique et les ellagitanins du même groupe sont constitués de polymère du glucose et des acides ellagique, gallique et/ou hexahydroxydiphénique.

En oenologie, on utilise des tanins pour le collage des vins car ils présentent une forte aptitude à se combiner aux protéines. Ils sont aussi utilisés pour améliorer les qualités organoleptiques et les caractères gustatifs des vins.

On emploie essentiellement des tanins extraits de châtaignier, de chêne et de noix de galles de divers arbres, qui sont des tanins hydrolysables.

L'application des tanins ne se limite pas à l'oenologie, mais elle s'étend à de nombreux autres domaines tels que ceux de la tannerie, de l'agroalimentaire, de la pharmacie, de la cosmétique, de la céramique, du textile...

Les tanins de raisin ne sont pas utilisés à l'échelle industrielle dans la fabrication des vins, en raison de la faible teneur en composés phénoliques des extraits obtenus. En effet les procédés actuellement utilisés pour préparer les tanins de raisin entraînent une dégradation importante desdits composés.

Malgré la faible proportion des composés polyphénoliques qu'ils renferment, on utilise les tanins de raisin et plus particulièrement de pépin de raisin, en pharmacie, à titre de médicament, notamment pour traiter des troubles liés à l'insuffisance veineuse. Le principe actif est la fraction des oligomères procyanidoliques contenus dans le tanin. Mais en raison de la grande dégradabilité de ces tanins, la pureté en dits oligomères des médicaments actuellement disponibles ne dépasse pas 20 %.

Le procédé d'obtention classiquement suivi pour obtenir des tanins, de raisin par exemple, consiste à : (a) effectuer, à partir de marc et/ou de pépin de raisin frais, noir et/ou blanc, une extraction solide-liquide d'une fraction brute de tanin dans un solvant aqueux, (b) éliminer le solvant aqueux de l'extrait résultant pour obtenir un concentré de la fraction brute de tanin, et (c) purifier la fraction brute de tanin pour obtenir ledit tanin, par une extraction liquide-liquide, une évaporation du solvant dans lequel le tanin est solubilisé puis une filtration sur charbon actif.

On apprend selon le document Chemical Abstracts, vol. 88, N°11 du 13/03/1978, Columbus, Ohio, US : abstract N°72940w, B. Mandzhukov : « Effect of some factors on the extraction of phenolic compounds from grapes » page 380, que l'addition de dioxyde de soufre (ou anhydride sulfureux), à une concentration de 50 mg/l, favorise l'extraction des anthocyanes, et à une concentration de 150 mg/l, favorise celle des tanins.

Ainsi le document FR-A-2 643 073 décrit un procédé d'obtention d'un extrait de composés phénoliques dénommés plenogénols du groupe des tanins procyanidines, ledit procédé comprenant les étapes suivantes :
on dispose de pépins de raisin à l'état brut,
on procède à une extraction solide-liquide par de l'eau qui peut être sulfitée (0,5 g/l sulfilte acide de sodium), à une température de 80°C,
après filtration à chaud, puis centrifugation du filtrat, on épuise le surnageant par de l'acétate d'éthyle selon une extraction liquide-liquide,
après séparation puis distillation de la fraction acétate d'éthyle, on lyophilise le résidu obtenu qui constitue l'extrait recherché.

D'autres documents enseignent des conditions d'obtention de tanins particulières permettant d'augmenter le rendement en tanins et leur pureté.

Selon l'abrégé N°1997-449638 accessible sur la base de données WP1, de la demande de brevet chinois CN A 1117485, on apprend que le prétraitement d'une solution de tanins galliques provenant de pommes de chêne, avec un colloïde à base d'aluminium et la purification des tanins ainsi traités par adsorption sur une résine macromoléculaire conduisent à l'obtention de tanins galliques de pureté élevée.

Selon l'abrégé de la demande de brevet chinois CN-A-1107885, on purifie des tanins polyphénoliques de vin par adsorption sur une résine de polyamide modifié puis élution à l'eau. Voir aussi WO86/06589.

Le brevet US-4735807 décrit un procédé d'obtention de vin à partir de céréales, mais les tanins ne sont pas isolés.

Selon la présente invention, on apporte un procédé d'obtention d'un tanin présentant une teneur élevée en composés polyphénoliques, ledit procédé incorporant des étapes déterminées de manière telle qu'elles permettent du préserver l'intégrité desdits composés.

Le procédé de l'invention est caractérisé en ce que, en combinaison, selon l'étape (a) on utilise comme solvant de l'eau sulfitée (H₂O + SO₂), et selon l'étape (c) on purifie la fraction brute de tanin par adsorption sélective des composes polyphénoliques du tanin sur résine et filtration consécutive.

- Avant d'exposer plus en détail la présente invention, divers termes employés dans la description et les revendications sont ci-après définis.

Le marc de raisin comprend la rafle du raisin c'est-à-dire la grappe sans ses grains (pédoncule et pédicelles) et la pellicule qui constitue l'enveloppe du grain de raisin.

Par tanin de marc de raisin, on entend donc le tanin obtenu par traitement du marc de raisin tel que défini ci-dessus. Mais, étant donné que les substances constitutives du tanin de marc proviennent en fait essentiellement de la pellicule du raisin, on devrait plus justement comprendre par cette expression, un tanin de pellicule de raisin. En oenologie, le marc est en fait une partie de la vendange récoltée pour la vinification, en blanc seulement, que l'on recueille après pressurage du jus de raisin puis séparation des pépins. C'est pourquoi il désigne de manière générique la pellicule. Ainsi selon le procédé de l'invention, quand la matière de départ est du marc de raisin frais, on comprendra qu'il peut s'agir de la pellicule seule de raisin frais.

Par raisin frais, on comprend Selon l'invention un raisin qui n'a subi ni fermentation alcoolique, ni piqûre acétique.

La nature des composés phénoliques totaux varie selon le type de tanin, comme précisé précédemment. Leur teneur est néanmoins toujours déterminée en équivalent d'acide gallique, selon la technique d'acide tanique du codex oenologique international.

Grâce au procédé de l'invention, on obtient un tanin comprenant notamment des gambiriines qui sont des dimères. Le tanin issu de pellicule et rafle selon le procédé décrit ci-dessus comprend en outre du resvératrol. Le tanin issu de la pellicule comprend aussi différents flavonols tels que la quercétine et le kaempférol.

Avantageusement, le procédé est défini par les caractéristiques supplémentaires suivantes, prises en combinaison ou non :
- l'étape (a) est effectuée à une température au plus égale à 25°C, et de préférence comprise entre 15 et 25°C,
- l'étape (b) est effectuée par évaporation sous vide à une température au plus égale à 75°C,
- après évaporation selon l'étape (b), le concentré de la fraction brute de tanin est immédiatement refroidi à une température au plus égale à 30°C,
- le procédé comprend une étape (b'), entre les étapes (b) et (c), selon laquelle on procède à une fermentation du concentré de la fraction brute de tanin ; aux fins de l'étape (b'), on ensemence des levures en suspension dans un milieu liquide, dans le concentré de la fraction brute de tanin ; on laisse la fermentation se dérouler ; on élimine l'alcool formé puis les particules en suspension pour obtenir un concentré de la fraction brute de tanin fermentée.

La fermentation a pour objet la transformation des sucres en alcool ainsi qu'une transformation bénéfique des tanins. Cette transformation rend les tanins conformes aux exigences organoleptiques des vins, car ils subissent le même processus de transformation que les tanins intrinsèques du vin.
- selon l'étape (c), la fraction brute de tanin est adsorbée sur une résine de type styrène divinylbenzène (DVB), de préférence microréticulé,
- selon l'étape (c), la fraction brute de tanin traitée sur résine est filtrée par diafiltration, en particulier diafiltration sélective ; cette dernière est avantageusement effectuée à une température au plus égale à 25°C.

On peut obtenir
- un tanin de marc de raisin noir et/ou blanc, comprenant une teneur en composés polyphénoliques totaux d'au moins environ 300 mg équivalent en acide gallique par g de matière sèche, ou
- un tanin de pépin de raisin noir et/ou blanc, comprenant une teneur en composés polyphénoliques totaux d'au moins environ 500 mg équivalent en acide gallique par g de matière sèche.

On peut utiliser un tanin de marc de raisin blanc tel que défini ci-dessus, comme tanin endogène du vin. On peut aussi utiliser un tanin de raisin pour obtenir un extrait riche en oligomères procyanidoliques.

La présente invention est maintenant illustrée à l'appui de l'exemple suivant.

### EXEMPLE :

### 1) Matière première

Selon cette illustration du procédé de l'invention, on a choisi du marc de raisin blanc, frais, pressé du jour. On peut utiliser du marc entier (rafles, pellicules, pépins) ou bien du marc éraflé (pellicules, pépins) plus ou moins pressé mais non dilacéré.
Le jus ou moût qui imprègne le marc ne doit avoir subi ni fermentation alcoolique, ni piqûre acétique.
Avantageusement, la pellicule est jaune à jaune verdâtre et doit surtout ne présenter aucune teinte brune, signe d'oxydations et de dégradations, et la rafle si elle est présente, est franchement verte.

### 2) Etape (a) d'extraction solide-liquide

### - Premier solvant :

Eau déminéralisée, sulfitée par 1 à 1,5 g d'anhydride sulfureux par litre.

### - Matériel et principes d'extraction

On utilise une batterie de diffusion qui se compose de 5 à 10 étages d'extraction et est décrite ci-après.
Elle constitue un alignement de cuves en béton, en forme de parallélépipède rectangle pouvant contenir de 10 à 50 tonnes de marc.
Un système de pompage aspire le liquide en fond de cuve et le rejette en surface du marc, ce qui permet de réaliser un lavage progressif, complet, mais non violent.
L'extrait résultant circule ensuite, d'une cuve à l'autre pour s'enrichir en composés polyphénoliques.

### - Paramètres d'extraction

### * Durée

Elle varie de 2 à 6 heures par cycle de lavage pour une même cuve, avant que l'extrait soit transféré dans la cuve suivante pour poursuivre son enrichissement en composés polyphénoliques.

Cette durée est déterminée en fonction de la richesse en composés polyphénoliques et de la structure du marc de départ.

### * Température

Elle varie de 15 à 25°C.
Une élévation serait préjudiciable à la qualité du tanin obtenu, et entraînerait l'extraction d'autres composés indésirables.
De plus, une température trop élevée favoriserait des pertes en SO₂ ainsi qu'un risque de fermentation des sucres présents en alcool. Cette formation d'alcool est particulièrement désavantageuse, car la moindre trace d'alcool va entraîner l'extraction de composés polyphénoliques indésirables, et au surplus très difficiles à éliminer ultérieurement;

### 3) Etape (b) d'élimination du premier solvant

### - Matériel

L'équipement destiné à concentration triple effet sous vide, est en Inox 316, et est muni d'un désulfiteur.
En sortie, on place un réfrigérant d'une surface suffisante pour atteindre rapidement une température inférieure à 30°C.

### - Principe

L'extrait obtenu en 2) [étape (a)] doit être ramené à une teneur en matière sèche (dont les sucres fermentescibles) qui correspond à une densité de 1,080 à 1,100 à 20°C, par élimination de l'eau et du SO₂, par évaporation sous vide à une température maximum de 75°C.
Le concentré de la fraction brute de tanin obtenu est aussitôt refroidi entre 26 et 28°C, pour l'étape (b') suivante de fermentation.

### 4) Etape (b') de fermentation

### - Ensemencement et fermentation

Le concentré obtenu en b) [étape (b)] est ensemencé par des levures oenologiques et des activateurs de fermentation.
La température en cours de fermentation ne dépasse de préférence pas 25°C.
Dès la fin de la fermentation, la fraction brute de tanin fermentée est refroidie à 15°C. Ce refroidissement permet d'atteindre une clarification suffisante par décantation en vue de l'élimination de l'alcool formé (ou désalcoolisation).

### - Elimination de l'alcool formé

Pour cette étape, on peut utilise le même matériel que celui décrit en 3).
Les paramètres de fonctionnement sont sensiblement les mêmes, à l'exception du débit d'alimentation qui sera inférieur, de manière à ne pas saturer le concentrateur avec les vapeurs d'alcool, et donc de bien récupérer celui-ci.
Le principe est identique à celui exposé en 3), sauf pour le contrôle de la matière sèche qui pour cette étape est suivi par indice de réfraction qui se situera entre 12 et 15 Brix.
La température de refroidissement en sortie concentrateur est de préférence comprise entre 10 et 13°C.
Le produit est maintenu sous agitation, le temps nécessaire à la formation des cristaux de tartrate de potassium, qui précipiteront ensuite par simple décantation et pourront être ainsi récupérés.

### - Elimination des particules en suspension

Les particules en suspensions qui consistent essentiellement en levures, cristaux de tartrate, floculats de polysaccharides, sont éliminées soit par centrifugation, soit par microfiltration.

### 5) Etape (c) de purification

Les deux techniques décrites ci-après sont employées consécutivement.

### 5.1) Purification par adsorption sélective des composés polyphénoliques du tanin

### - Matériel

On utilise des colonnes de résine en acier ébonité, garnies de résine adsorbante de type Styrène DVB, telle que l'Amberlite XAD 16 MP commercialisée par Rohm and Haas.

### - Principe

Le traitement du concentré de la fraction brute de tanin fermenté obtenu en 4), avec le matériel décrit ci-dessus, comprend deux étapes :
* une phase de fixation sélective des composés polyphénoliques par adsorption sur la résine, et donc élimination des composés autres que les composés polyphénoliques, puis
* une phase d'élution des composés phénoliques adsorbés par une solution d'éthanol à 50°GL.

### 5.2) Purification par séparation sur membrane des composés polyphénoliques

### - Matériel

On utilise un module de nanofiltration équipé de membranes organiques d'un seuil de coupure de 100 à 300 daltons.

### - Principe

L'éluat (composés polyphénoliques dans éthanol) obtenu en 5.1) est désalcoolisé et concentré par rétention des composés polyphénoliques sur la membrane.
Il est préférable d'utiliser la technique de diafiltration car elle permet de maximaliser la désalcoolisation.
Les paramètres de fonctionnement seront alors de 8 à 10 bars et de 20°C maximum.
Le perméat résultant (eau, éthanol et quelques sels) pourra être recyclé pour l'élution de 5.1) par distillation classique.

Après purification, l'éluat concentré ci-dessus (ou rétentat) qui présente une teneur en matières sèches de 10 à 15 %, est ensuite séché, par atomisation classique avec les paramètres suivants :
* température air entrée tour: 190°C
* température air sortie tour : 85°C
Ce procédé permet d'obtenir une poudre fine, fluide peu pulvérulente.

Pour obtenir une poudre granulée, on utilisera une tour d'atomisation de type M.S.D. avec les paramètres suivants :
* température air entrée tour: 190°C
* température air sortie tour : 85°C
* température plaque/poudre : 69°C
* température lit statique : 75°C
Cette technique de séchage permet d'obtenir facilement des granulés réguliers.

## Revendications

1. Procédé d'obtention d'un tanin de *pellicule* et/ou de pépin de raisin, comprenant les étapes consistant à (a) effectuer, à partir de *pellicule* de raisin frais et/ou de pépin de raisin frais, noir et/ou blanc, une extraction solide-liquide d'une fraction brute de tanin dans *de l'eau sulfitée* (H₂O+SO₂), (b) éliminer le solvant aqueux de l'extrait résultant pour obtenir un concentré de la fraction brute de tanin, et (c) purifier la fraction brute de tanin pour obtenir ledit tanin,
**caractérisé en ce que,** selon l'étape (c) on purifie la fraction brute de tanin par adsorption sélective des composés polyphénoliques du tanin sur résine et filtration consécutive.

2. Procédé selon la revendication 1, **caractérisé en ce que** l'étape (a) est effectuée à une température au plus égale à 25°C, et de préférence comprise entre 15 et 25°C.

3. Procédé selon la revendication 1, **caractérisé en ce que** l'étape (b) est effectuée par évaporation sous vide à une température au plus égale à 75°C.

4. Procédé selon la revendication 3, **caractérisé en ce que** après évaporation, le concentré de la fraction brute de tanin est immédiatement refroidi à une température au plus égale à 30°C.

5. Procédé selon la revendication 1, **caractérisé en ce qu'**il comprend une étape (b'), entre les étapes (b) et (c), selon laquelle on procède à une fermentation du concentré de la fraction brute de tanin.

6. Procédé selon la revendication 5, **caractérisé en ce qu'**aux fins de l'étape (b'), on ensemence des levures en suspension dans un milieu liquide, dans le concentré de la fraction brute de tanin ; on laisse la fermentation se dérouler ; on élimine l'alcool formé puis les particules en suspension pour obtenir un concentré de la fraction brute de tanin fermentée.

7. Procédé selon la revendication 1, **caractérisé en ce que** selon l'étape (c) la fraction brute de tanin est adsorbée sur une résine de type styrène DVB.

8. Procédé selon la revendication 1, **caractérisé en ce que** selon l'étape (c) la fraction brute de tanin traitée sur résine est filtrée par diafiltration.

## Claims

1. A method for producing a grape skin and/or seed tannin, comprising the steps consisting of (a) carrying out, starting with fresh black and/or white grape skin and/or seed, a solid-liquid extraction of a crude tannin fraction in sulphited water (H₂O+SO₂), (b) removing the aqueous solvent from the resulting extract in order to obtain a concentrate of the crude tannin fraction, and (c) purifying the crude tannin fraction in order to obtain the tannin,
**characterized in that**,
in step (c), the crude tannin fraction is purified by selective adsorption of the polyphenolic compounds of the tannin on resin and subsequent filtration.

2. The method according to claim 1, **characterized in that** the step (a) is carried out at a temperature of 25°C or less, and is preferably comprised between 15 and 25°C.

3. The method according to claim 1, **characterized in that** the step (b) is carried out by evaporation under vacuum at a temperature at most equal to 75°C.

4. The method according to claim 3, **characterized in that**, after evaporation, the concentrate of the crude tannin fraction is immediately cooled to a temperature at most equal to 30°C.

5. The method according to claim 1, **characterized in that** it comprises a step (b'), between steps (b) and (c), in which the concentrate of the crude tannin fraction is fermented.

6. The method according to claim 5, **characterized in that** step (b') is carried out by inoculating yeasts in suspension in a liquid medium, into the concentrate of the crude tannin fraction; allowing to proceed fermenting; removing the formed alcohol and the particles in suspension in order to obtain a concentrate of the fermented crude tannin fraction.

7. The method according to claim 1, **characterized in that** in step (c), the crude tannin fraction is adsorbed on the resin which is of the styrene DVB type.

8. The method according to claim 1, **characterized in that** in step (c), the crude tannin fraction which is treated on resin, is further filtered by diafiltration.

## Patentansprüche

1. Verfahren zur Herstellung eines Tannins aus Traubenhaut und/oder Traubenkernen, die Schritte umfassend, die darin bestehen, (a) aus der Haut und/oder den Kernen frischer roter und/oder weißer Trauben einen festflüssigen Rohanteil Tannin in Sulfitwasser (H₂O + SO₂) zu extrahieren, (b) die wässrige Lösung des resultierenden Extrakts abzuscheiden, um ein Konzentrat des Tannin-Rohanteils zu erhalten, und (c) den Tannin-Rohanteil zu reinigen, um das besagte Tannin herzustellen, **dadurch gekennzeichnet, dass** der Tannin-Rohanteil gemäß Schritt (c) durch selektive Adsorption der Polyphenolverbindungen des Tannins über Harz und nachfolgende Filtration gereinigt wird.

2. Verfahren nach Anspruch 1, **dadurch gekennzeichnet, dass** der Schritt (a) bei einer Temperatur durchgeführt wird, die höchstens gleich 25 °C beträgt und vorzugsweise zwischen 15 und 25 °C liegt.

3. Verfahren nach Anspruch 1, **dadurch gekennzeichnet, dass** der Schritt (b) durch Vakuumverdampfung bei einer Temperatur von höchstens gleich 75 °C durchgeführt wird.

4. Verfahren nach Anspruch 3, **dadurch gekennzeichnet, dass** das Konzentrat des Tannin-Rohanteils nach der Verdampfung sofort auf eine Temperatur von höchstens gleich 30 °C abgekühlt wird.

5. Verfahren nach Anspruch 1, **dadurch gekennzeichnet, dass** es zwischen den Schritten (b) und (c) einen Schritt (b') umfasst, dem gemäß eine Fermenterung des Konzentrats des Tannin-Rohanteils durchgeführt wird.

6. Verfahren nach Anspruch 5, **dadurch gekennzeichnet, dass** das Konzentrat des Tannin-Rohanteils am Ende von Schritt (b') mit in einem wässrigen Milieu suspendierten Hefen beimpft wird, die Fermentierung abläuft, der gebildete Alkohol und danach die Schwebeteilchen abgezogen werden, um ein Konzentrat des fermentierten Tannin-Rohanteils zu erhalten.

7. Verfahren nach Anspruch 1, **dadurch gekennzeichnet, dass** der Tannin-Rohanteil gemäß Schritt (c) auf einem Harz vom Typ Styren DVB adsorbiert wird.

8. Verfahren nach Anspruch 1, **dadurch gekennzeichnet, dass** der auf Harz behandelte Tannin-Rohanteil gemäß Schritt (c) durch Diafiltration filtriert wird.
